# EUROPEAN PATENT APPLICATION

(11) **EP 0 724 860 A1**
(43) Date of publication of application: **07.08.1996**
(21) Application number: 96101609.4
(22) Date of filing: 05.02.1996
(51) Int. Cl.: A61B 5/024

(54) **Self-aligning photoplethysmograph sensor**

(30) Priority: 03.02.1995 US 383106
(71) Applicant: SPACELABS MEDICAL, INC., Redmond, Washington 98073 (US)
(72) Inventor: Hartwig, Robert W., Tacoma, Washington 98422 (US)
(74) Representative: Grünecker, Kinkeldey, Stockmair & Schwanhäusser Anwaltssozietät

(57) **Abstract**

A self-aligning photoplethysmograph sensor (30) includes a flexible, resilient web loop (32) having a circumference smaller than the circumference of a body part on which the photoplethysmograph sensor is to be used. The loop (32) is formed by an inner loop of a resilient material covered by an outer loop of fabric. A red light emitting diode (34), an infrared light emitting diode (30), and a light detector (40) are mounted on the inner surface of the inner loop with the light detector directly opposite the light emitting diodes. As a result, when the loop is placed around a finger (F), light emanating from the light emitting diodes shines through the finger and is incident on the light detector. A pair of conductive leads (44,48) bonded to the inner surface of the inner loop extends in a serpentine manner from each of the light emitting diodes and the light detector to a cable junction (46) on the outer loop. A cable (50) has a respective plurality of wires that are connected at one end to the conductive leads and at the other end to respective pins (54) of a cable connector (52) that is mounted on the other end of the cable.

## Description

### Technical Field

This invention relates to photoplethysmograph sensors, and more particularly to a transmissive photoplethysmograph sensor that inherently places its light emitter in alignment with its light detector.

### Background of the Invention

Photoplethysmograph sensors are electro-optical devices that are commonly used in the medical field to sense a variety of physiological parameters. Photoplethysmograph sensors use a light emitter optically coupled to patient tissues, and a light detector that receives the emitted light after it passes through the tissues. A common photoplethysmograph sensor is a pulse oximetry sensor that uses both a red light emitting diode and an infrared light emitting diode, both of which are coupled to a photodetector through vascularized tissues. The photodetector generates signals that are indicative of the absorption of red and infrared light in the vascularized tissues, and these signals are used to determine the oxygen saturation of a patient's arterial blood.

There are basically two types of photoplethysmograph sensors, including two types of pulse oximetry sensors. These sensor types are reflective sensors and transmissive sensors. Reflective sensors direct light from a light emitter into the tissues, and the photodetector determines the amount of light reflected from the tissues. Thus, reflective sensors generally utilize light emitters and light detectors that are positioned adjacent to each other on substantially the same plane. The other type of photoplethysmograph sensors are transmissive sensors which direct light from a light emitter to the light detector through the patient tissues. As a result, transmissive sensors use a light emitter and a light detector that face each other from two spaced apart locations.

There are at least two varieties of transmissive photoplethysmograph sensors in common use. The first is a "clip-on" sensor in which two pivotally interconnected legs of a clip are resiliently biased toward each other. One of the legs of the clip carries one or more light emitters, and the other leg of the clip carries a light detector. The clip-on transmissive sensor is clipped onto a patient's body part, such as a finger or ear, so that the light emitter faces the light detector with the body part therebetween. The principle disadvantage of clip-on transmissive sensors is that they are generally somewhat heavy and bulky, thus making them somewhat obtrusive to wear. Furthermore, the compressive force applied to the body part can become uncomfortable after they are worn for a long period of time. Finally, since clip-on transmissive sensors are held in position only by friction, they can easily become dislodged as the patient moves.

The other commonly used variety of transmissive sensor uses a strip of adhesive having one or more light emitters mounted on a surface at one location and a light detector mounted on the same surface at a different location. An example of a "stick on" photoplethysmograph sensor is described and shown in U.S. Patent No. 4,830,014 to Goodman et al. Stick on transmissive sensors are used by wrapping the adhesive strip around or over the end of the finger of a patient with the light emitter(s) positioned opposite the light detector. Optimum sensor performance is achieved when the light detector is aligned directly opposite the light emitter(s). Assistance in aligning the light emitter(s) with the light detector is provided by markings on the outer surface of the adhesive strip adjacent to both the light emitter(s) and the light detector.

In theory, the adhesive strip is applied to the finger of a patient with the alignment markings positioned opposite each other thereby ensuring alignment of the light emitter(s) with the light detector. In practice, it is often not possible or practical to apply the adhesive strip in this manner for several reasons. First, the stick on sensors are often placed on a patient in emergency conditions where the medical practitioner does not have the time to carefully apply the adhesive strip to the patient who may be in motion during the application procedure. Second, if the medical practitioner attaches either more than or less than one-half of the strip to one side of the patient's finger, then there will be either too much or too little adhesive strip available to attach to the other side of the patient's finger. Under these circumstances, it will not be possible to place the alignment marks opposite each other without removing the adhesive strip from the patient's finger and starting over. Third, stick on sensors are commercially available only with spacings between the light emitters and the light detector of 20 and 25 mm. This fixed spacing precludes the sensor from being in proper alignment any time the sensor is installed on a finger that does not have a circumference of twice this spacing, i.e., 40 or 50 mm. Finally, while the alignment marks may be positioned opposite each other so that axial alignment is proper, the light emitter(s) and light detector may be misaligned from each other in a transverse direction. Thus, conventional stick on transmissive photoplethysmograph sensors are often not attached to the patient with the light emitter(s) properly aligned with the light detector.

Even if the stick on photoplethysmograph sensors have been properly attached to the patient, the light emitter(s) and light detector can subsequently become misaligned. After the patient has moved about over a considerable period of time, the adhesive strip can creep because of the nature of the adhesive so that the initial alignment becomes altered. Also, the strength of the adhesive can deteriorate with time and patient movement thus eventually allowing the stick on photoplethysmograph sensor to detach from the patient's finger. To prevent these problems, medical practitioners have a tendency to use external means to secure the sensor in place. However, the use of these external securing means can cut off blood flow and cause pressure necrosis in underlying tissues. These problems can also be caused by applying the stick on sensors too tightly. If the sensor has not been attached to the skin for such a long period that the strength of the adhesive has deteriorated, removal of the sensor from the patient, especially neonates, can tear the skin.

Other problems associated with stick on photoplethysmograph sensors are caused by the adhesive residue itself. First, adhesive residue invariably remains after the photoplethysmograph sensor has been removed from the finger of the patient. Second, sterilization agents in the adhesive residue and the adhesive itself may produce an allergic reaction when the sensor is attached to the skin.

For the above reasons, conventional stick on photoplethysmograph sensors do not adequately solve the problem of attaching a transmissive photoplethysmograph sensor to a patient in a manner that not only ensures initial proper alignment, but also ensures that the alignment remains proper during use.

### Summary of the Invention

It is an object of the invention to provide a photoplethysmograph sensor that can be installed correctly even by relatively untrained individuals.

It is another object of the invention to provide a photoplethysmograph sensor that can be installed correctly without exercising a great deal of care and attention.

It is another object of the invention to provide a self-aligning photoplethysmograph sensor that is readily adaptable to a variety of sensor functions, such as pulse oximetry.

It is still another object of the invention to provide a transmissive photoplethysmograph sensor that does not use adhesive.

It is a further object of the invention to provide a self-aligning photoplethysmograph sensor that is relatively inexpensive and is comfortable to wear due to its relatively light weight and small size.

These and other objects of the invention are provided by a photoplethysmograph sensor formed by a resilient web loop having an inner surface on which a light detector and a light emitter, such as a light emitting diode, are mounted substantially opposite each other. As a result, the light emitter is positioned opposite the light detector when the loop is stretched around a body part, such as a finger, with an inner surface of the loop in contact with the body part. The photoplethysmograph sensor may include a second light emitter mounted on the inner surface of the loop adjacent the first light emitter so that the first and second light emitters are equidistant from the light detector, and thus both aligned with the light detector, when the loop is placed around the body part. In the event that the first and second light emitters emits red and infrared light, respectively, the photoplethysmograph sensor may be used as a pulse oximetry sensor. The flexible, resilient web loop is preferably formed by an inner loop of a resilient material having the light emitter and the light detector mounted on its inner surface, and the inner loop is then surrounded by an outer loop of fabric. The photoplethysmograph sensor preferably also includes at least one conductive lead extending in a serpentine manner from the light emitter and from the light detector to a cable junction, and a cable connected at one end to the outer loop at the cable junction. The cable has a plurality of respective wires connected at one end to the conductive leads and at the other end to respective pins of a cable connector.

### Brief Description of the Drawings

Figure 1 is an isometric view of a prior art transmissive photoplethysmograph sensor.

Figure 2 is an isometric view of a presently preferred embodiment of the inventive self-aligning photoplethysmograph sensor.

Figure 3 is a cross-sectional view taken along the line 3-3 of Figure 2.

Figure 4 is a side elevational view of the sensor of Figure 2.

### Detailed Description of the Invention

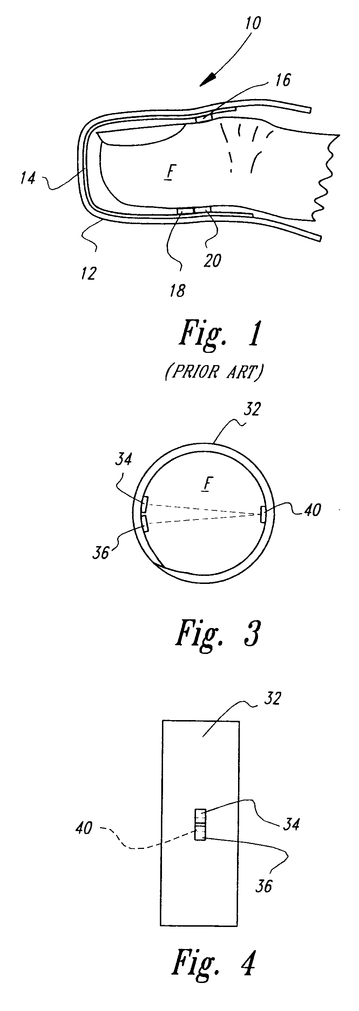

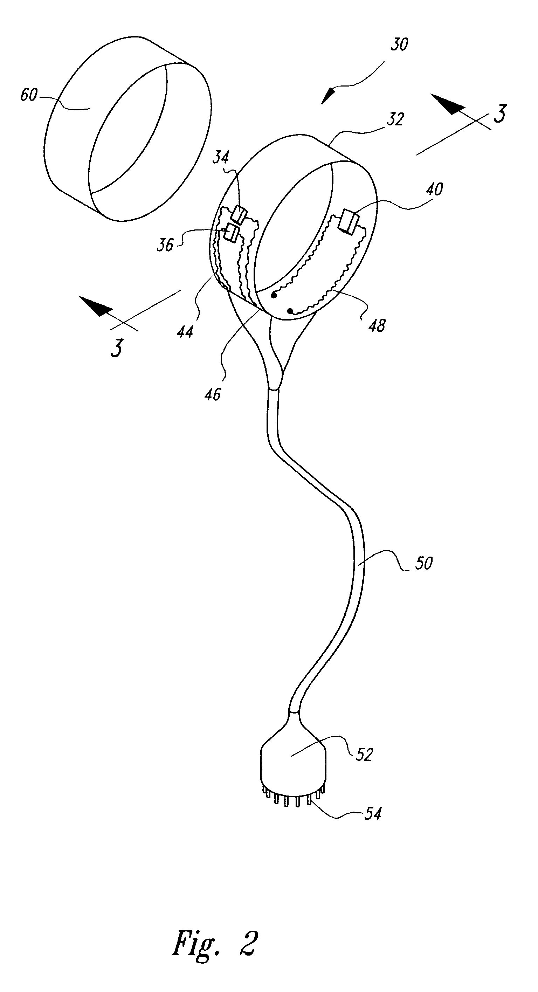

A prior art "stick on" transmissive photoplethysmograph sensor 10 is illustrated in Figure 1. The photoplethysmograph sensor 10 is a pulse oximetry sensor of the type described in U.S. Patent No. 4,830,014 to Goodman et al. The sensor 10 includes a flexible strip 12 having an inner surface coated with a layer of adhesive 14. The strip 12 has the appearance of a conventional Band-Aid®. A photodetector 16 is mounted on the same surface of the strip 12 and a pair of light emitters 18, 20 are also mounted on the inner surface of the strip 12 at a location spaced apart from the light detector 16.

Although not shown in Figure 1, the sensor 10 may also be used by wrapping it around the finger F circumferentially so that it is perpendicular to the position shown in Figure 1. However, the above-mentioned problems with the sensor 10 also exist when it is used in this manner.

In use, the sensor 10 is placed on the finger F of a patient with the light emitters 18, 20 positioned opposite the light detector 16. By aligning the light emitters 18, 20 with the light detector 16, the maximum quantity of light emitted by the light emitters 18, 20 passes through the finger F of the patient to the light detector 16. The outside surface of the strip 12 contains an alignment (not shown) marking opposite the light detector 16 and a similar alignment marking (not shown) opposite the light emitters 18, 20.

As explained above, it is often difficult in practice to ensure that the sensor 10 is placed on the finger F with the light emitters 18, 20 in proper alignment with the light detector 16. Even if the light emitters 18, 20 are properly aligned with the light detector 16 in the longitudinal direction, as shown in Figure 1, the light emitters 18, 20 may be transversely offset from the light detector 16. Under these circumstances, the quantity of light passing from the light emitters 18, 20 to the light detector 16 will not be optimal. Also, the nature of the adhesive 14 allows the strip 12 to creep during use thus altering the relative position between the light emitters 18, 20 and the light detector 16. Finally, when the sensor 10 is removed from the finger F, a residue of adhesive 14 is invariably left on the surface of the finger F.

A preferred embodiment of the inventive self-aligning transmissive photoplethysmograph sensor 30 is best illustrated in Figure 2. The photoplethysmograph sensor 30 includes a loop 32 of resilient material, such as, for example, a cloth weave containing elastic thread. The circumference of the loop 32 is slightly less than the circumference of a body part on which the sensor 30 is to be placed. As illustrated in Figure 2, the sensor 30 is adapted to be placed around the finger of a patient. A red light emitting diode 34 and an infrared light emitting diode 36 of conventional design are mounted on the inner surface of the loop 32. A photodetector 40 of conventional design is mounted on the inner surface of the loop 32 opposite the light emitting diodes 34, 36. Thus, the light emitting diodes 34, 36 are equally spaced from the light detector 40. The light emitting diodes 34, 36 are attached to respective pairs of conductive leads, generally indicated at 44 which extend from the light emitting diodes 34, 36 to a cable junction location 46 on the outer surface of the loop 32. Similarly, a pair of leads, generally indicated at 48, extend from the light detector 40 to the cable junction location 46. The leads 44, 48 run in a serpentine manner so that they will not break as the loop 32 is stretched for a placement around a finger, as described below. The leads 44, 48 may be secured to the loop 32 by any suitable means such as by weaving the leads 44, 48 through the loop (in the event that the loop 32 is formed by a weave) or by bonding the leads 44, 48 to the inner or outer surfaces of the loop 32. The leads 44, 48 are connected to respective wires (not shown) in a sensor cable 50 that extends from the cable junction location 46 to a sensor connector 52 having a plurality of pins 54 which are connected to respective wires (not shown) extending through the cable 50. The sensor connector 52 is connected to a conventional pulse oximetry monitor which applies appropriate signals to the light emitting diodes 34, 36 through the connector 52 and cable 50 and receives signals from the light detector 40 through the cable 50 and connector 52.

Although not required, the loop 32 is preferably covered by a cylindrical cloth jacket 60 which may be adhesively bonded to the loop 32.

It will be apparent from an examination of Figure 2 that, when the loop 32 is placed around a finger, the light emitting diodes 34, 36 will inherently be aligned with the light detector 40. In this manner, the optimum amount of light from the light emitters 34, 36 will be picked up by the light detector 40. As a result, it is not necessary to use a great deal of care in installing the sensor 30 on a patient, and alignment will be proper even in emergency conditions and/or where the patient is in motion. Furthermore, the light emitters 34, 36 will remain in alignment with the light detector 40 even after prolonged use of the sensor 30. Finally, since the loop 32 is not adhesively bonded to the skin of the patient, an adhesive residue is not left on the skin of the patient after the sensor 30 has been removed.

Although the sensor 30 is shown in Figure 2 as a pulse oximetry sensor having red and infrared light emitting diodes, 34, 36, respectively, it will be understood that the inventive self-aligning photoplethysmograph sensor can be used for other purposes. For example, a photoplethysmograph used to monitor the pulse rate of a patient would have a single light emitter and a single light detector. Also, the sensor may be used to monitor any blood constituent lending itself to non-invasive measurement.

The manner in which the self-aligning photoplethysmograph sensor 30 inherently ensures alignment of the light emitters 34, 36 with the light detector 40 is illustrated in Figure 3. As shown therein, the loop 32 is placed around the finger F of a patient. The geometry of the light emitters 34, 36 relative to the light detector 40 ensures that the light emitting diodes 34, 36 are longitudinally aligned with the light detector 40. Furthermore, as illustrated in Figure 4, the geometry of the light emitting diodes 34, 36 relative to the light detector 40 ensures that the light emitting diodes 34, 36 are aligned transversely with the light detector 40.

Although the inventive sensor is shown in use on a finger, it will be understood that it may also be used on other body parts including neonate wrists, neonate ankles, neonate palms, neonate feet, neonate arms and legs, and toes, to name a few.

It is thus seen that the inventive transmissive photoplethysmograph sensor ensures proper alignment both initially and after subsequent use even though it is placed on the finger of a patient without a great degree of care. Although the sensor 30 is shown for installation on a finger, it will be understood that it may also be easily adapted for installation on other body parts. It will also be evident that, although specific embodiments of the invention have been described herein for purposes of illustration, various modifications may be made without deviating from the spirit and scope of the invention.

## Claims

1. A self-aligning photoplethysmograph sensor, comprising:
a resilient web loop having a circumference smaller than the circumference of a body part on which said photoplethysmograph sensor is to be used so that said loop stretches when said loop is placed around the body part with an inner surface in contact with the body part;
a first light emitter mounted on an inner surface of said loop so that light emanating from said light emitter is incident on the body part when said loop is placed around the body part; and
a light detector mounted on an inner surface of said loop substantially opposite from said light emitter so that segments of said loop between said light emitter and said light detector are of substantially equal length whereby when said loop is placed around a body part light shining through the body part from the light emitter is incident on the light detector.

2. The self-aligning photoplethysmograph sensor of claim 1 further including a second light emitter on the inner surface of said loop adjacent said first light emitter, said first and second light emitters being positioned substantially equidistant from said light detector.

3. The self-aligning photoplethysmograph sensor of claim 2 wherein said first light emitter emits red light, and said second light emitter emits infrared light whereby said sensor may be used as a pulse oximetry sensor.

4. The self-aligning photoplethysmograph sensor of claim 1 wherein said resilient web loop comprises:
an inner loop of a resilient material having said light emitter and said light detector mounted on an inner surface thereof; and
an outer loop of fabric surrounding to said inner loop.

5. The self-aligning photoplethysmograph sensor of claim 4, further comprising:
a respective pair of conductive leads extending in a serpentine manner from said light emitter to a cable junction and from said light detector to said cable junction;
a cable connected at one end to said outer loop at said cable junction, said cable having a plurality of respective wires connected to said conductive leads; and
a cable connector connected to the other end of said cable, said cable connector having a plurality of conductive pins connected to respective wires of said cable.

6. The self-aligning photoplethysmograph sensor of claim 1, further comprising:
a respective pair of conductive leads extending through said loop in a serpentine manner from said light emitter to a cable junction and from said light detector to said cable junction;
a cable connected at one end to said loop at said cable junction, said cable having a plurality of respective wires connected to said leads; and
a cable connector connected to the other end of said cable, said cable connector having a plurality of conductive pins connected to respective wires of said cable.

7. A self-aligning pulse oximetry sensor, comprising:
a resilient web loop having a circumference smaller than the circumference of a body part on which said photoplethysmograph sensor is to be used so that said loop stretches when said loop is placed around the body part with an inner surface in contact with the body part;
a red light emitting diode mounted on an inner surface of said loop so that light emanating from said red light emitting diode is incident on the body part when said loop is placed around the body part;
an infrared light emitting diode mounted on an inner surface of said loop adjacent said red light emitting diode so that light emanating from said infrared light emitting diode is incident on the body part when said loop is placed around the body part;
a light detector mounted on an inner surface of said loop substantially equidistant from each of said light emitting diodes so that light from the light emitting diodes shines through the body part and is incident on the light detector when said loop is placed around the body part;
at least one conductive lead extending through said loop in a serpentine manner from a cable junction to each of said light emitting diodes and to said light detector;
a cable connected at one end to said loop at said cable junction, said cable having a plurality of respective wires connected to said conductive leads; and
a cable connector connected to the other end of said cable, said cable connector having a plurality of conductive pins connected to respective wires of said cable.

8. The self-aligning pulse oximetry sensor of claim 7, wherein said resilient web loop comprises:
an inner loop of a resilient material having said light emitting diodes and said light detector mounted on an inner surface thereof; and
an outer loop of fabric surrounding to said inner loop.

9. A method of determining the transmissivity of light through a body part, comprising:
providing a loop of resilient material having a circumference smaller than the circumference of the body part
mounting a first light emitter on an inner surface of said loop
mounting a light detector on an inner surface of said loop substantially opposite from said light emitter so that segments of said loop between said light emitter and said light detector on opposite sides of said light detector are of substantially equal length; and
stretching said loop and placing it around the body part with the inner surface of the loop in contact with the body part so that light emanates from said light emitter, passes through the body part, and is incident on the light detector.

10. The method of claim 9 further including the step of mounting a second light emitter on the inner surface of said loop adjacent said first light emitter with said first and second light emitters positioned substantially equidistant from said light detector.

11. The method of claim 9 wherein said loop has a circumference that is slightly smaller than the circumference of a human finger, and wherein said step of stretching said loop and placing it around the body part comprises stretching said loop and placing it around the finger.
